# EUROPÄISCHE PATENTSCHRIFT

(11) **EP 2 175 808 B1**
(45) Veröffentlichungstag und Bekanntmachung des Hinweises auf die Patenterteilung: **18.12.2013**
(21) Anmeldenummer: 08773958.7
(22) Anmeldetag: 10.07.2008
(51) Int. Cl.: A61F 2/58, A61F 2/68

(54) **PROTHETISCHE GREIFEINHEIT**
PROSTHETIC GRIP UNIT
UNITÉ DE PRÉHENSION PROTHÉTIQUE

(30) Priorität: 30.07.2007 DE 102007035965
(43) Veröffentlichungstag der Anmeldung: 21.04.2010
(73) Patentinhaber: Otto Bock Healthcare Products GmbH, 1070 Wien (AT)
(72) Erfinder: PUCHHAMMER, Gregor, A-1130 Wien (AT)
(74) Vertreter: Stornebel, Kai
(86) Internationale Anmeldenummer: PCT/EP2008/005636
(87) Internationale Veröffentlichungsnummer: WO 2009/015751

(56) Entgegenhaltungen:
- EP-A- 0 261 276
- EP-A- 1 277 451
- US-A- 2 573 351
- US-A- 4 114 464
- US-A- 4 685 925
- US-A- 4 808 187
- US-A- 5 013 326

## Beschreibung

Die Erfindung betrifft eine prothetische Greifeinheit mit einem Grundkörper, an dem Anschlussmittel zur Befestigung an einer oberen Extremität angeordnet sind, von dem zumindest zwei Ausleger abstehen, von denen ein erster Ausleger relativ zu dem zweiten Ausleger bereeglich aus einer abstehenden in eine anliegenden Stellung verlagerbar gelagert ist. Die Erfindung betrifft insbesondere so genannten Hooks, die eine einfache und stabile Greifeinrichtung darstellen. Die Schwenkachse des beweglichen Auslegers ist dabei senkrecht zum Grundkörper ausgerichtet, der die Mittelhand einer natürlichen Hand nachbildet.

Aus dem Stand der Technik sind solche prothetischen Greifeinheiten seit langer Zeit bekannt, beispielsweise aus der GB 2 109 245 A1 beschrieben. Um den beweglich an dem Grundkörper gelagerten Ausleger in Richtung auf einen feststehenden, zweiten Ausleger zu bewegen, ist an dem proximalen Ende der beiden Ausleger ein Gummiband angeordnet, dass so dimensioniert ist, dass die beiden Ausleger in eine anliegende Stellung gebracht werden. Über einen Bowdenzug wird der beweglich gelagerte Ausleger in eine abgespreizte Stellung verlagert. Durch die Federkraft wird der Ausleger in Richtung auf den starren Ausleger bewegt und kann dort Gegenstände festhalten. Diese mechanisch einfachen und robusten Greifeinrichtungen haben den Nachteil, dass die Griffkraft durch die Vorspannung des elastischen Bandes limitiert ist. Weiterhin müssen relativ hohe Kräfte durch den Träger der prothetischen Greifeinheit aufgebracht werden, um den beweglichen Ausleger abzuspreizen.

Die US 4,685,925 beschreibt eine prothetische Greifeinheit mit zwei Hakenelementen, die an einem Grundkörper gelagert sind. Ein Hakenelement steht fest, das andere Hakenelement ist schwenkbar gelagert und wird über ein elastisches Band in Richtung auf das feststehende Hakenelement vorgespannt. Über einen Elektromotor kann das schwenkbar gelagerte Hakenelement geöffnet werden. US 4685925 Offenbart den Oberbegriff des Anspruch 1 der Anmeldung.

Die US 4,114,464 beschreibt eine Kunsthand mit zumindest einem Finger und einem Daumen, der mit einem Antriebsmotor und einem Getriebe gekoppelt ist, um den Finger und den Daumen aufeinander zu und voneinander weg zu bewegen. Ein Schneckengetriebe ist vorgesehen, um eine Kraftübertragung von dem Motor auf die Finger zu realisieren. Die Schließkraft oder Schließgeschwindigkeit kann über einen Elektromagneten geändert werden, der eine Kupplungsscheibe schaltet.

Die EP 261 276 A1 betrifft eine myoelektrisch gesteuerte Kunsthand mit einem Fingerpaar, das schwenkbar an einem Grundkörper gelagert ist. Elektromotoren sind in dem Gehäuse angeordnet, um eine Relativverlagerung der Finger zu bewirken.

Die US 5,013,326 betrifft eine Prothesenhand mit zwei beweglich zueinander gelagerten Hakenelementen.

Aufgabe der vorliegenden Erfindung ist es, eine prothetische Greifeinheit bereitzustellen, die für den Prothesennutzer angenehmer zu benutzen ist.

Erfindungsgemäß wird diese Aufgabe durch eine prothetische Greifeinheit mit den Merkmalen des Anspruchs 1 gelöst. Vorteilhafte Ausgestaltungen und Weiterbildungen der Erfindung sind in den Unteransprüchen aufgeführt.

Die prothetische Greifeinheit mit einem Grundkörper, an dem Anschlussmittel zur Befestigung an einer oberen Extremität angeordnet sind, von dem zumindest zwei Ausleger abstehen, von denen ein erster Ausleger relativ zu dem zweiten Ausleger beweglich aus einer abstehenden in eine anliegende Stellung verlagerbar gelagert und deren Schwenkachse senkrecht zu dem Grundkörper ausgerichtet ist, sieht vor, dass der erste Ausleger motorisch angetrieben ist. Durch die motorische Aktuierung des beweglich gelagerten Auslegers ist es möglich, unabhängig von der elastischen Vorspannung durch ein Gummiband eine schnelle und kontrollierte Betätigung durchzuführen, so dass eine hohe Griffkraft unabhängig von der Federvorspannung erreicht werden kann. Dadurch ist es für den Nutzer nicht mehr notwendig, eine entsprechend hohe Öffnungskraft aufzubringen, um mit einer ausreichend hohen Griffkraft einen Gegenstand fassen zu können. Der motorische Antrieb erlaubt eine schnelle Aktuierung sowie eine wahlweise Schaltung der Greifeinrichtung in einer offenen oder geschlossenen Ruhestellung, Eine platzsparende Ausgestaltung der Erfindung sieht vor, dass der Antrieb in dem Grundkörper und der erste Ausleger auf dem Lager des Antriebes gelagert ist, um so ein möglichst großes und stabiles Lager für den beweglichen Ausleger bereitstellen zu können. Um grundsätzliche Einstellungen für die Schließkraft oder die Geschwindigkeit der Schwenkbewegung vornehmen zu können, sind Einstelleinrichtungen an dem Grundkörper oder an der Energiespeichereiheit vorgesehen, die bevorzugt an der Rückseite der Greifeinheit, die dem Handrücken entspricht, angeordnet sind, um eine leichte Einstellung durch den Prothesennutzer bewirken zu können. Der Antrieb ist reversibel ausgebildet, um ein Öffnen und Schließen der Greifeinheit ermöglichen zu können. Einstelleinrichtungen oder Einstellvorrichtungen für eine Modusumschaltung können ebenfalls vorgesehen sein, wobei diese Einrichtungen eine Umschaltung zwischen verschiedenen Steuerungsarten ermöglichen, beispielsweise zwischen einer Kraftregelung, einer Positionsregelung, einer EVO- (electronic voluntary opening) oder einer EVC-Steuerung (electronic voluntary closing). Die Einstelleinrichtungen können sowohl die Modusumschaltung als auch die Kraft- oder Geschwindigkeitseinstellungen bewirken.

Sofern der zweite Ausleger starr an dem Grundkörper befestigt oder einstückig daran ausgebildet ist, wird ein einfacher konstruktiver Aufbau dadurch erreicht, dass nur ein Ausleger mit dem Antrieb gekoppelt werden muss. Grundsätzlich ist jedoch auch vorgesehen, dass der zweite Ausleger motorisch angetrieben ist, so dass beide Ausleger zueinander beweglich gelagert sind.

Um die jeweiligen Ausleger an den gewünschten Einsatzzweck anpassen oder um beschädigte Ausleger austauschen zu können, sind diese austauschbar an dem Grundkörper befestigt, beispielsweise eingeschraubt oder über einen Karabinerverschluss daran befestigt. Dadurch ist es möglich, für den jeweiligen Einsatzzweck geeignete Ausleger anzubringen.

Der Grundkörper ist bevorzugt als das Gehäuse für den Antrieb ausgebildet, so dass innerhalb des Grundkörpers der Antrieb angeordnet ist. Als Antrieb ist insbesondere ein Elektromotor vorgesehen, der unmittelbar mit dem beweglich gelagerten Ausleger verbindbar oder verbunden ist. Ebenfalls kann der beweglich gelagerte Ausleger über ein Getriebe mit dem Antrieb bzw. Elektromotor gekoppelt sein, um eine gewünschte Übersetzung und dadurch eine Anpassung entweder der Verstellgeschwindigkeit oder der Greifkraft herbeiführen zu können.

Die Ausleger können hakenförmig ausgebildet sein, um eine hohe Variabilität der Einsatzmöglichkeiten bereitstellen zu können. Ein dritter Ausleger kann starr an dem ersten Ausleger befestigt oder daran angeformt sein, um eine zusätzliche Abstützmöglichkeit für gegriffene Gegenstände bereitzustellen. Ein solcher dritter Ausleger ist insbesondere zur Abstützung eines Stiftes oder eines anderen Werkzeuges vorgesehen, der oder das zwischen den beiden Auslegern eingeklemmt ist. Dabei ist der erste Ausleger bevorzugt zwischen dem zweiten und dem dritten Ausleger angeordnet und steht seitlich, beispielsweise in einem rechten Winkel, von dem ersten Ausleger ab. Der dritte Ausleger kann ebenfalls abgewinkelt sein, um ein Abrutschen des aufgelegten Gegenstandes zu vermeiden.

An dem Grundkörper ist eine Aufnahme für eine Speichereinrichtung für elektrische Energie angeordnet, so dass leicht ein Austausch einer Energiespeichereinheit vorgenommen werden kann. Die Energiespeichereinheit, beispielsweise eine wiederaufladbare Batterie, kann leicht an dem Grundkörper angekoppelt werden, beispielsweise über einen Schraubmechanismus oder einen Karabinerverschluss, so dass eine Wechselbatterie bzw. ein Wechselakku schnell getauscht werden kann.

Durch die motorische Aktuierung des beweglich gelagerten Auslegers ist es möglich, diesen über myoelektrische Impulse anzasteuern, wozu eine entsprechende myoelektrische Ansteuerung des Antriebes vorgesehen ist. Die Betätigung der Greifeinheit benötigt somit keine ausladende Schulterbewegung, sondern kann über myoelektrische Impulse erfolgen. Um den Antrieb, insbesondere den Motor auf der Grundlage myoelektrischer Impulse betreiben zu können, ist in dem Grundkörper eine Steuerungselektronik für den Antrieb angeordnet, bevorzugt eingekapselt, so dass nur noch eine Kopplung mit den Elektroden oder myoelektrischen Impulsgebern zu erfolgen hat, um die prothetische Greifeinheit in Betrieb zu nehmen. Die Steuerung kann auch in dem Energiespeicher angeordnet sein oder innerhalb des Antriebes liegen.

Sowohl die Steuereinheit als auch der Antrieb sind wasserdicht gekapselt, um auch bei Benutzung der prothetischen Greifeinheit in einer rauen Umgebung die Funktionssicherung zu gewährleisten.

Um eine möglichst kompakte Bauweise zu erreichen, ist die Antriebsachse des Antriebes bzw. Motors parallel, insbesondere koaxial zu der Schwenkachse des ersten Auslegers orientiert, so dass eine unmittelbare Kopplung ohne Umlenkgetriebe möglich ist. Sollte eine Getriebestufe zwischengeschaltet werden müssen, kann diese leicht koaxial angeordnet und ausgebildet werden.

Um bei einem Ausfall des Motors bzw. der Energieversorgung die Funktionalität weiterhin zu gewährleisten, ist der Antrieb von dem ersten Ausleger entkoppelbar ausgebildet. Zur Bereitstellung der notwendigen Griffkraft kann dann ein herkömmliches Kraftspeicherelement, beispielsweise in Form eines elastischen Bandes, um die Ausleger herum gelegt werden. Dazu sind an dem ersten Ausleger und dem zweiten Ausleger Halteeinrielitungen für ein entsprechendes Federelement ausgebildet, beispielsweise Aufnahmen für einen elastischen Ring oder für eine Zugfeder.

An dem dritten Ausleger kann in einer Weiterbildung der Erfindung ein Betätigungshebel gelagert sein, der über einen längsverschieblich gelagerten Bowdenzug mit dem Träger der prothetischen Greifeinheit gekoppelt ist. Über diesen Betätigungshebel kann in für den Prothesennutzer gewohnter Art und Weise eine Aktuierung der Ausleger erfolgen, wenn keine myoelektrische Kopplung vorgesehen ist. Die längsverschiebliche Lagerung ermöglicht eine Anpassung an die körperlichen Gegenebenheiten des Prothesennutzers. Die Aktuierung erfolgt dabei in der Regel über eine Schulterbewegung, so dass ein Prothesennutzers mit einem großen Bewegungsradius eine Anordnung des Bowdenzuges an dem distalen Ende des dritten Auslegers bevorzugt, während bei einer Bewegungseinschränkung oder bei einem geringen Bewegungsunfang aufgrund der physiologischen Gegebenheiten eine proximale Anordnung des Bowdenzuges an dem Hebel zu wählen wäre.

Eine Weiterbildung der Erfindung sieht vor, dass zumindest eine Sensoreinheit zur Erfassung des Betätigungselementes des Betätigungshebels vorhanden und mit Betätigungshebel gekoppelt ist, so dass in Abhängigkeit von der Betätigungskraft und der über den Betätigungshebel ausgeübten Betätigungsmomente eine Steuerung der Griffkraft erfolgen kann. Je höher die Betätigungskräfte sind, desto größer wird die Griffkraft eingestellt, so dass eine angepasste Griffkraft zwischen den beiden aneinander liegenden Auslegern eingestellt werden kann. Ebenfalls kann ein Sensor zur Ermittlung der aktuellen Griffkraft vorhanden sein, der eine Rückmeldung an die Steuereinheit übermittelt und den Antrieb sperrt oder für eine Aufrechterhaltung der Griffkraft sorgt.

Zur Entkupplung des ersten Auslegers mit dem Antrieb ist der erste Ausleger über eine verlagerbare Verzahnung mit dem Antrieb gekoppelt, so dass bei Außereingriffbringen der Verzahnung mit dem Antrieb eine Verstellung unabhängig von dem Antrieb möglich ist.

In einer Weiterbildung der Erfindung ist vorgesehen, dass der Antrieb einen Kraftgang und einen Schnellgang aufweist, zwischen denen mechanisch oder elektrisch umgeschaltet werden kann. Dadurch kann zunächst mit einer hohen Übersetzung unter Aufbringung einer relativ geringen Greifkraft ein schnelles Verfahren zweier Ausleger aufeinander zu ermöglicht werden. Treten die Ausleger in Kontakt mit dem zu greifenden Objekt, wird die Griffkraft automatisch erhöht, während die Greifgeschwindigkeit verringert wird. Diese Umschaltung kann rein mechanisch über einer entsprechende Schaltkupplungsanordnung oder über eine elektronische Steuerung erfolgen.

Ausführungsbeispiele der Erfindung werden in den nachfolgenden Figuren erläutert. Gleiche Bezugszeichen in den Figuren bezeichnen gleiche oder gleichwirkende Bauelemente. Es zeigen:
- Figur 1 -: eine Teilschnittansicht einer Greifeinrichtung;
- Figur 2 -: eine perspektivische Schrägdraufsicht einer ersten Variante;
- Figur 3 -: eine perspektivische Schrägdraufsicht einer zweiten Variante;
- Figur 4 -: eine Greifeinrichtung gemäß Figur 2 mit nicht montierter Energiespeichereinrichtung;
- Figur 5 -: eine perspektivische Schräguntenansicht;
- Figur 6 -: eine Variante der Figur 2 mit einem Entriegelungsmechanismus; sowie
- Figur 7 -: eine Schnittdarstellung der Lagerung.

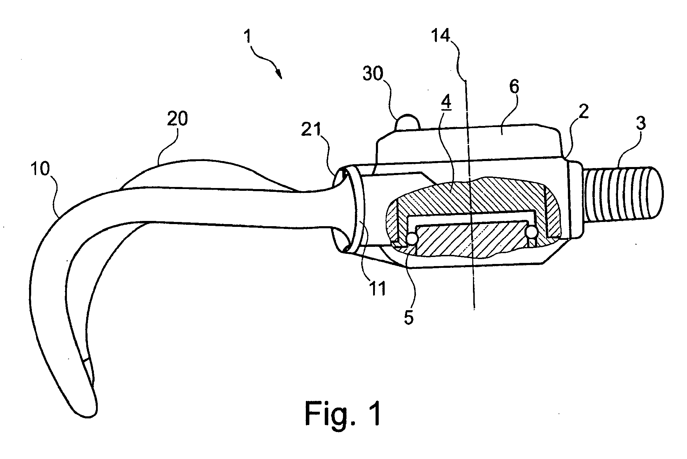

In der Figur 1 ist in einer teilgeschnittenen Darstellung eine prothetische Greifeinheit 1 mit einem Grundkörper 2 dargestellt, der bevorzugt aus einem Metall, insbesondere Leichtmetall hergestellt ist. An dem Grundkörper 2 ist ein Anschlussmittel 3 in Gestalt eines Gewindes als Normanschluss für die prothetische Greifeinheit an beispielsweise einer Unterarmprothese oder an eine Aufnahmevorrichtung vorgesehen, die an einem Unterarm befestigt ist. Die Anschlussmittel 3 sind insbesondere als Schraubgewinde ausgebildet. Die Anschlussmittel 3 können eine abweichende Ausgestaltung aufweisen, beispielsweise als Bajonettverschluss oder dergleichen.

Innerhalb des Gehäuses 2 ist ein Antrieb 4 in Gestalt eines flachbauenden Elektromotors angeordnet. Der Grundkörper 2 bildet einen Rahmen oder ein Chassis sowohl für das Anschlussmittel 3 auch für zwei Ausleger 10, 20, von denen im dargestellten Ausführungsbeispiel der erste Ausleger 10 schwenkbar an dem Grundkörper 2 gelagert ist. Der zweite Ausleger 20 ist fest an dem Grundkörper 2 befestigt, insbesondere angeschraubt oder in eine Aufnahme eingeführt und daran gesichert. Der Antrieb 4 als Elektromotor ist innerhalb des Grundkörpers 2 über ein Lager 5 gelagert, das gleichzeitig das zentrale Lager für den beweglichen Ausleger 10 darstellt.

Alternativ zu einer festen Ausgestaltung des zweiten Auslegers 20 kann dieser ebenfalls über den Antrieb 4 angetrieben sein und relativ auf den ersten Ausleger 10 bewegt werden. Die Schwenkbewegung erfolgt dabei um die Schwenkachse 14, die senkrecht zu der Grundfläche des Antriebes 4 verläuft und bevorzugt parallel, insbesondere koaxial zu der Drehachse des Antriebes 4 verläuft. Die Schwenkachse 14 verläuft dabei senkrecht zu derjenigen Fläche, die bei einer natürlichen Hand der Handfläche bzw. dem Handrücken entsprechen würde. Die beiden Ausleger 10, 20 können in eine offene Stellung oder in eine aneinander anliegende Stellung verfahren werden, um in der aneinander anliegenden Stellung Objekte greifen zu können bzw. zwischen sich einzuklemmen. Bevorzugt sind die Ausleger 10, 20 an ihrer distalen Enden gekrümmt und somit als Haken ausgebildet, da sich diese Geometrie als besonders geeignet für einfache Greifverrichtungen erwiesen hat.

Auf der Oberseite des Grundkörpers 2 ist eine Batterie 6 bzw. ein Akkumulator befestigt, der austauschbar an dem Grundkörper 2 gelagert ist. An dem auswechselbaren Akkumulator 6 können Einstelleinrichtungen vorgesehen sein, über die die Schnelligkeit der Verstellung ebenso wie die aufzubringende Griffkraft zwischen den Auslegern 10, 20 einstellbar ist. Das Bezugszeichen 30 bezeichnet eine Spitze eines dritten Auslegers, der in der Perspektive von dem Hauptantrieb 4 verdeckt wird.

An den proximalen Enden der Ausleger 10, 20 sind Vorsprünge 11, 21 ausgebildet, die als Barrieren für ein elastisches Band 8 dienen, so dass dieses sicher an den Auslegern 10, 20 gehalten und gegen ein Abrutschen in distale Richtung gesichert wird.

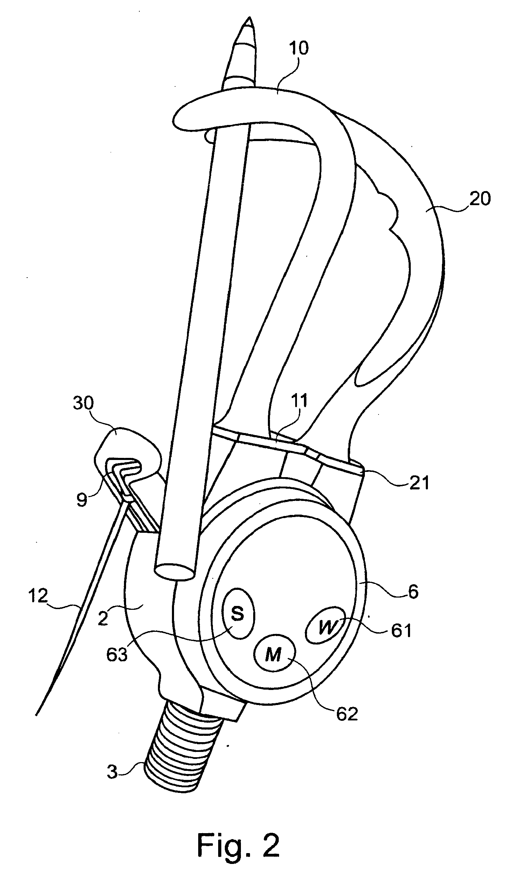

In der Figur 2 ist in einer perspektivischen Schrägdraufsicht die Greifeinheit 1 dargestellt, in der zu erkennen ist, dass an dem ersten Ausleger 10 ein dritter Ausleger 30 angeordnet ist, der die Funktion des Daumens bzw. eines Auflagers für einen zu greifenden Gegenstand, in dem dargestellten Ausführungsbeispiel eines Stiftes, ausübt. Der dritte Ausleger 30 ist zusammen mit dem ersten Ausleger 10 verschwenkbar an dem Grundkörper 2 gelagert, bevorzugt bildet die Aufnahme für den ersten Ausleger 10 zusammen mit dem dritten Ausleger 30 ein einstückiges Bauteil. Alternativ kann der dritte Ausleger 30 auch eingeschraubt oder eingesetzt sein.

Der zweite Ausleger 20 steht in dem dargestellten Ausführungsbeispiel fest und ist austauschbar an dem Grundkörper 2 befestigt.

Das Anschlussmittel 3 kann gegen ein myoelektrisches Anschlussstück ausgetauscht werden, bei dem eine zusätzliche Verkabelung zu dem Antrieb 4 bereitgestellt werden muss. Bei einem myoelektrischen Anschlussstück wird die Verkabelung bevorzugt durch das Anschlussmittel 3 hindurchgeführt.

Auf der Oberseite der prothetischen Greifeinheit 1 und dort auf der Oberseite des Energiespeichers 6 sind Sensorflächen 61, 62, 63 als Einstelleinrichtungen für die maximal aktive Schließkraft der Ausleger 10, 20 und gegebenenfalls der Schließgeschwindigkeit vorhanden.

Innerhalb des dritten Auslegers 30 ist ein Betätigungshebel 9 angeordnet, der über einen Bowdenzug 12, der verschieblich und feststellbar an dem Betätigungshebel 9 gelagert ist, aktuiert wird. Der Bowdenzug 12 ist an dem Oberarm oder der Schulter des Prothesennutzers angeordnet und bewirkt bei einer Schulterbetätigung eine Aktuierung des Motors 4 und dadurch ein Schließen der Ausleger 10, 20 bzw. ein Öffnen. Das Öffnen kann bei einer wiederholten Betätigung des Bowdenzuges 12 erfolgen. Dazu ist es vorgesehen, dass der Antrieb 4 reversibel ausgebildet ist.

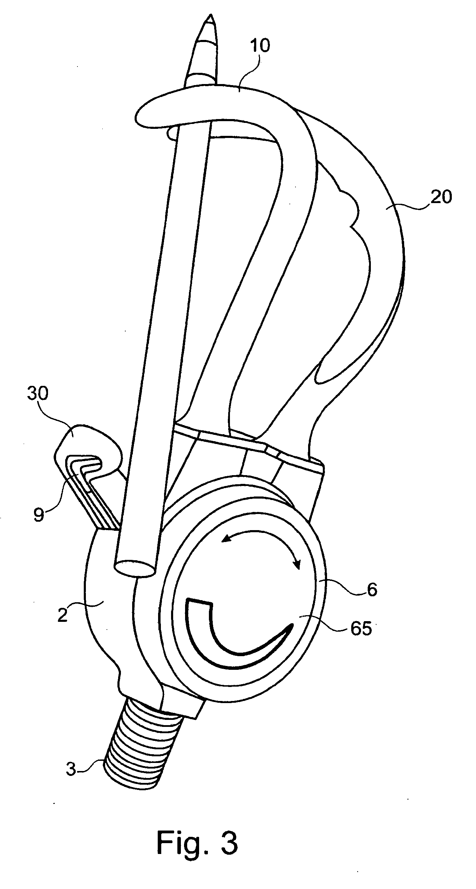

Eine Variante der Ausführungsform gemäß Figur 2 ist in der Figur 3 dargestellt, bei der statt punktförmiger Sensorfelder 61, 62, 63 eine Einstellung durch eine drehbare Abdeckkappe 65 erfolgt. Die Drehbarkeit in beide Richtungen ist durch den Doppelpfeil angedeutet. Bei einer Verdrehung im Uhrzeigersinn wird die Griffkraft erhöht, bei einer Drehung entgegen dem Uhrzeigersinn wird die Griffkraft verringert.

Weiterhin können auf der Rückseite des Akkumulators 6 weitere Sensorfelder oder Einstellmöglichkeiten angeordnet sein, beispielsweise für die Verstellgeschwindigkeiten oder eine Umschaltung der Steuerungsmodi. Ebenfalls ist es möglich, statt auf der Rückseite bzw. Oberseite der Greifeinheit 1 die Sensorfelder seitlich an dem Gehäuse 2 angeordnet werden.

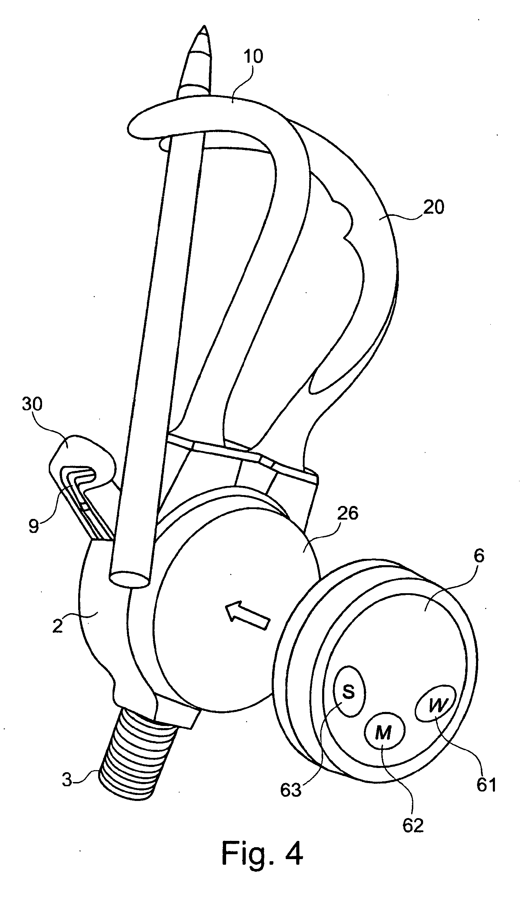

In der Figur 4 ist eine Variante der Figur 2 dargestellt, bei der die Speichereinrichtung 6 für elektrische Energie als eine austauschbare Kapsel auf der Rückseite der Greifeinheit 1 an einer entsprechend ausgestalteten Aufnahme 26 angebracht werden kann. Die Fixierung der Speichereinrichtung 6 erfolgt durch Aufschrauben oder durch alternative, insbesondere formschlüssige Befestigungsarten. Durch die Austauschbarkeit der Speichereinrichtung 6 kann die Unabhängigkeit des Prothesennutzers vergrößert werden, da längere Betriebsdauern durch einen einfachen und schnellen Wechsel des Akkumulators 6 ermöglicht werden kann. Der Akkumulator 6 selbst ist wasserdicht ausgeführt und hat eine Kontaktierung zu dem Antrieb 4, die ebenfalls wasserdicht ausgebildet ist. Ebenfalls kann eine weitere Sensorik auf der Rückseite der Greifeinheit 1 bzw. in dem Akkumulator 6 angeordnet sein, wobei durch eine Anordnung des Akkumulators 6 auf der Rückseite die Sicht auf den jeweils gegriffenen Gegenstand kaum beeinträchtigt wird.

Durch die Integration des Akkumulators 6 in die Greifeinheit 1 kann ein autonomes Greifsystem bereitgestellt werden, das leicht gegen ein herkömmliches, mechanisches Greifsystem ausgetauscht werden kann. Die Anordnung des Bowdenzuges 12 kann so gestaltet sein, dass ein Neujustierung oder Ablenkung der Züge nicht notwendig ist, so dass ein einfacher Austausch der motorisch angetriebenen Greifeinheit 1 gegen herkömmliche Systeme leicht möglich ist.

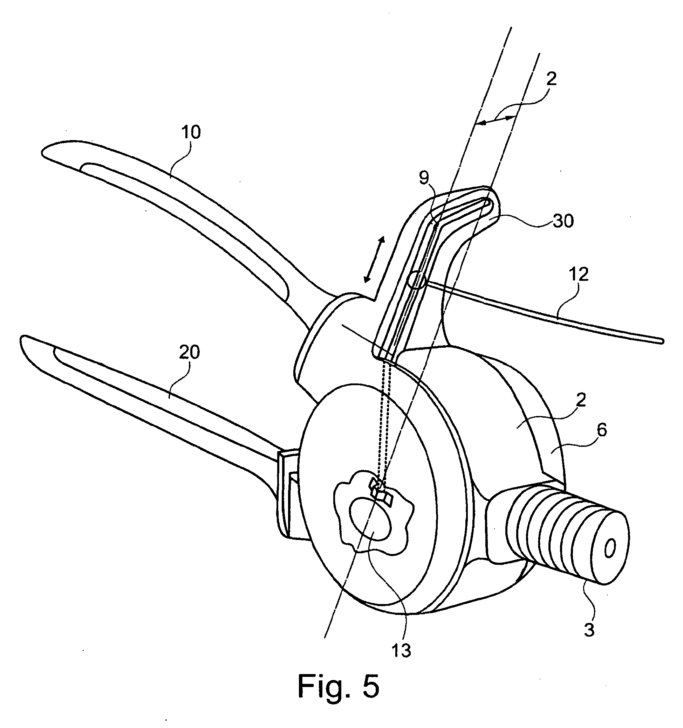

In der Figur 5 ist die Unterseite der Greifeinheit 1 dargestellt, anhand der der Betätigungshebel 9 und dessen Funktionsweise erkannt werden kann. Der Bowdenzug 12 kann über ein ballförmiges Abschlussstück oder Klemmeinrichtungen verschieblich an dem Betätigungshebel 9 gelagert sein, wie dies durch den Doppelpfeil angedeutet ist. An dem Betätigungshebel 9 ist also eine verschiebliche Festlegung des Bowdenzugs 12 gegeben. An der Unterseite des Gehäuses 2 oder innerhalb des Gehäuses 2 ist eine Sensoreinrichtung 13 angeordnet, die das Betätigungsmoment an dem Betätigungshebel 9 misst. Der Hebelarm L, der durch den Betätigungshebel 9 bzw. den Angriffspunkt des Bowdenzuges 12 entlang seines Verschiebeweges ausgebildet wird, ermöglicht es, dass das Sensorsignal des Sensors 13, das durch eine Zugkraft innerhalb des Bowdenzuges 12 entsteht, unabhängig von der Position des Endstückes des Bowdenzuges 12 an dem Betätigungshebel 9 ist. Dadurch ist es möglich, ein Sensorsignal von der Sensoreinheit 13 bereitzustellen, das proportional zur Zugkraft des Bowdenzuges 12 ist. Der Angriffspunkt des Bowdenzuges 12 kann somit an einer beliebigen Stelle innerhalb des Verschiebebereiches gewählt werden, wobei die Aufnahme des Bowdenzuges 12 in dem Anschlussstück bzw. der Klemmvorrichtung so gewählt ist, dass ein schneller Austausch der motorisch angetriebenen Greifeinheit 1 gegen eine konventionelle Greifeinheit möglicht ist. Der zweite Lagerungspunkt des Betätigungshebels 9 ist an dem nach oben abgekrümmten Ende des dritten Auslegers 30, im so genannten Daumen.

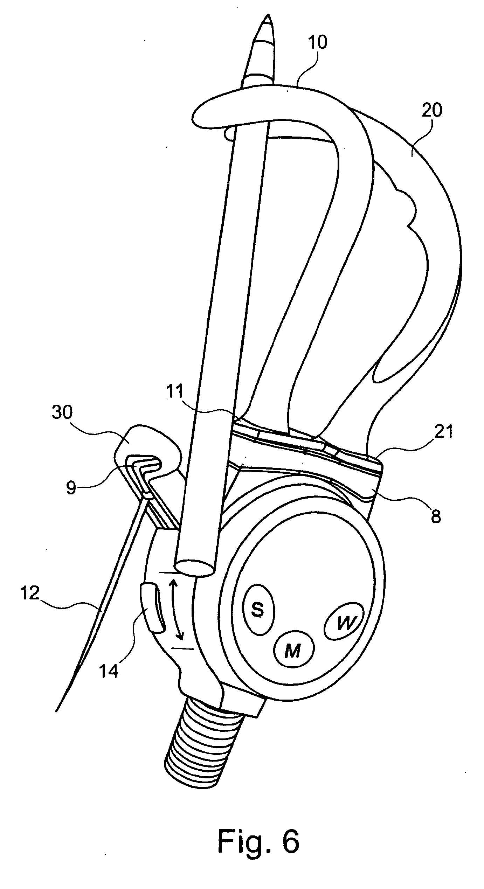

In der Figur 6 ist eine Variante der Erfindung gezeigt, bei der eine Sicherheitseinrichtung integriert ist, die bei einer elektrischen Fehlfunktion der prothetischen Greifeinheit ein manuelles Öffnen der Ausleger 10, 20 ermöglicht. Durch ein Verschieben der Einstelleinrichtung 14 wird eine Planverzahnung im Inneren des Antriebes 4 außer Eingriff gebracht, so dass der beweglich gelagerte Ausleger 10 und damit der dritte Ausleger 30 freigegeben wird. Das Betätigungselement 14 ist in beide Verschieberichtungen wirksam, so dass das Verschieben auch beidseitig erfolgen kann. Sofern eine Entkopplung der schwenkbar gelagerten Ausleger 10, 30 von dem Antrieb 4 erfolgt ist, kann eine freie Beweglichkeit realisiert werden. Um ein Schließen der Ausleger 10, 20 zu ermöglichen, ist ein Federelement 8 in Gestalt eines elastischen Ringes bzw. eines elastischen Bandes vorgesehen, das als Notlösung zur Bereitstellung der Schließkraft montiert wird. Die Vorsprünge 11, 21 verhindern ein Abrutschen von den Auslegern 10, 20. Über den Bowdenzug 12 und den entsprechend stabil ausgestalteten Betätigungshebel 9 kann die Greifeinrichtung 1 auch ohne Energieversorgung bzw. bei einer elektrischen Störung betätig werden, so dass ein Notbetrieb erfolgt, wenn auch dadurch die erfindungsgemäßen Vorteile verloren gehen.

Innerhalb des Gehäuses 2 können Steuereinrichtungen für eine myoelektrische Ansteuerung angeordnet sein, die Steuereinrichtung kann auch separat innerhalb des Akkumulators 6 integriert sein. Die Einstellung der Griffkraft kann mittels eines Potentiometers erfolgen, ebenfalls kann darüber die Schnelligkeit der Schließ- bzw. Öffnungsbewegung erfolgen. Alternativ dazu ist eine mechanische Steuerung über die Betätigung des Betätigungshebels 9 und eine Schulteraktuierung vorgesehen.

Über Sensoren kann die jeweilige Stellung der Ausleger 10, 20, 30 ermittelt werden, so dass bei einem anliegenden myoelektrischen Signal eine korrekte Zuordnung der Drehbewegung des Antriebes 4 erfolgen kann. Die Ruhestellung der Greifeinheit kann frei gewählt werden, entweder offen, mit auseinander bewegten Auslegern 10, 20, oder geschlossen. Über eine propriozeptorische Sensorik ist es möglich, eine angepasste Griffkraft bereitzustellen. Während übliche mechanische Griffeinheiten durch eine Kraft am Bowdenzug gegen eine Federkraft geöffnet werden, kann dies relativ einfach elektromotorisch über ein einfaches Signal erfolgen. Die rein mechanische Öffnung der Greifeinheit benötigt ein Zweifaches bis Vierfaches derjenigen Kraft an Zugkraft an dem Bowdenzug, die an der Spitze der Ausleger 10, 20 verfügbar ist. Der Betätigungsweg ist daher entsprechend klein und wird über einen Schulterzug aufgebracht. Die erreichbare Griffkraft bei einer rein mechanischen Lösung wird durch die Stärke der Feder bzw. des Gummibandes und die Hebelübersetzung definiert und ist festgelegt. Durch die motorische Antreibbarkeit und eine kraftabhängige Steuerung ist es möglich, die an den Auslegern zu erzeugenden Kräfte an die Betätigungskräfte anzupassen, in ihrer Höhe jedoch zu entkoppeln. Größere Kräfte auf den Betätigungshebel 9 führen zu einer verstärkten Schließkraft, das Niveau der Schließkraft kann jedoch unabhängig von der Stärke der Betätigungskraft eingestellt werden. Dadurch wird die Betätigungskraft von dem Betätigungsweg entkoppelt, da eine Sensorik zur Messung der Kraft innerhalb des Bowdenzuges 12 vorhanden ist. Dies ist für eine Anpassung an den Patienten wichtig, da die unterschiedlichen physiologischen Gegebenheiten berücksichtigt werden können. Der Betätigungsweg für die vollständige Öffnung der Ausleger 10, 20 wird durch die Position des Bowdenzuges 12 an dem Betätigungshebel 9 bestimmt. Für kleine Personen oder Personen mit einer eingeschränkten Beweglichkeit wird ein Anschlusspunkt in der Nähe des ersten Auslegers 10 gewählt, also nahe der Drehmitte, für große Personen mit einer großen Beweglichkeit in dem Schulterbereich ist ein möglichst distaler Anschlusspunkt für den Bowdenzug vorzusehen.

Eine entsprechende Steuerung kann vorgesehen sein, bei der sich die Greifeinheit grundsätzlich bis zum Erreichen der eingestellten Kraft bzw. des eingestellten Momentes schließt. Diese eingestellte Kraft kann um ein Vielfaches höher liegen als es bei den bisherigen mechanischen Greifeinheiten üblich oder möglich ist.

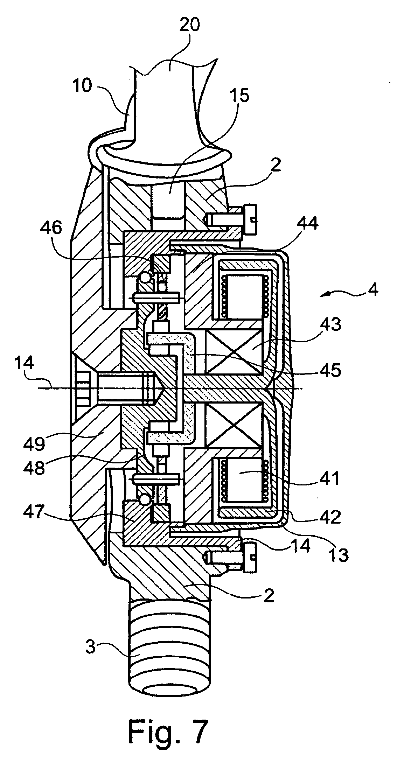

In der Figur 7 ist in einer Schnittdarstellung die Lagerung des beweglichen Auslegers 10 an dem Grundkörper 2 gezeigt. Innerhalb des Grundkörpers 2 ist der gesamte Antrieb 4 mit einem Motor gelagert, der einen Stator 41 und einen Rotor 42 aufweist. Eine Abdeckkappe 13 deckt den Antrieb 4 nach außen ab. Der Rotor 42 dreht sich um die Schwenkachse 14 und ist an einer Rotorlagerung 43 gelagert. Innerhalb des Grundköpers 2 und des Antriebs 4 ist eine Motor- und Steuerungselektronik 44 angeordnet, über die verschiedene Bewegungsmodi oder Geschwindigkeiten bzw. Griffkräfte eingestellt werden können. Über einen Exzenter 45 mit Kupplungsteil zum Rotor 42 ist der Rotor mit einer Zykloidenscheibe 46 verbunden, die über Stifte mit einer Abtriebscheibe 48 gekoppelt ist. Die Abtriebscheibe 48 ist an dem Außenring 47 des Hauptlagers gelagert und mit einer Scheibe 49 verbunden, die beweglich zu dem Grundkörper 2 gelagert ist und an der der bewegliche Ausleger 10 befestigt ist. Die Scheibe 49 ist an der Abtriebscheibe 48 über eine Schraube gesichert. Das Hauptlager 47 ist über eine Halterung 14, die über Schrauben an dem Grundkörper 2 gesichert ist, in dem Grundköper 2 gehalten. Die Halterung 14 drückt dabei auf den Lageraußenring des Hauptlagers 47.

Am oberen Ende der Figur 7 ist zu erkennen, dass der feststehende Ausleger 20 über einen Zapfen 15 an dem Grundkörper 2 befestigt ist, beispielsweise eingeschraubt oder eingeklemmt. Der Antrieb 4 und der bewegliche Ausleger 10 sind in dem dargestellten Ausführungsbeispiel gemeinsam über das Hauptlager 47 an dem Grundkörper 2 gelagert.

## Patentansprüche

1. Prothetische Greifeinheit (1) mit einem Grundkörper (2), an dem Anschlussmittel (3) zur Befestigung an einer oberen Extremität angeordnet sind, von dem zumindest zwei Ausleger (10, 20) abstehen, von denen ein erster Ausleger (10) relativ zu dem zweiten Ausleger (20) beweglich aus einer abstehenden in eine anliegende Stellung verlagerbar gelagert ist und die Schwenkachse (14) senkrecht zum Grundkörper (2) ausgerichtet ist, wobei der erste Ausleger (10) motorisch angetrieben ist, **dadurch gekennzeichnet, dass** der Antrieb (4) in dem Grundkörper (2) gelagert ist, dass der erste Ausleger (10) auf dem Lager (5) des Antriebes (4) gelagert ist und dass an dem Grundkörper (2) eine Einstelleinrichtung (61, 62, 63, 65) für die Schließkraft und/oder Schnelligkeit der Schwenkbewegung angeordnet ist.

2. Prothetische Greifeinheit nach Anspruch 1, **dadurch gekennzeichnet, dass** dein dritter Ausleger (30) starr an dem ersten Ausleger (10) befestigt oder daran angeformt ist.

3. Prothetische Greifeinheit nach Anspruch 1 oder 2, **dadurch gekennzeichnet, dass** an dem Grundkörper (2) eine Aufnahme (26) für eine Speichereinrichtung (6) für elektrische Energie angeordnet ist.

4. Prothetische Greifeinheit nach einem der voranstehenden Ansprüche, **gekennzeichnet durch** eine myoelektrische Ansteuerung des Antriebes (4).

5. Prothetische Greifeinheit nach einem der voranstehenden Ansprüche, **dadurch gekennzeichnet, dass** in dem Grundkörper (2) eine Steuerungselektronik für den Antrieb (4) angeordnet ist.

6. Prothetische Greifeinheit nach einem der voranstehenden Ansprüche, **dadurch gekennzeichnet, dass** eine Steuerungselektronik in dem Antrieb (4) angeordnet ist.

7. Prothetische Greifeinheit nach einem der voranstehenden Ansprüche, **dadurch gekennzeichnet, dass** der Antrieb (4) wasserdicht gekapselt ist.

8. Prothetische Greifeinheit nach einem der voranstehenden Ansprüche, **dadurch gekennzeichnet, dass** die Antriebsachse (14) des Antriebes (4) parallel, insbesondere koaxial zur Schwenkachse des ersten Auslegers (10) orientiert ist.

9. Prothetische Greifeinheit nach einem der voranstehenden Ansprüche, **dadurch gekennzeichnet, dass** an dem Grundkörper (2) eine Einstelleinrichtung (61, 62, 63, 65) für eine Modusumschaltung angeordnet ist.

10. Prothetische Greifeinheit nach einem der voranstehenden Ansprüche, **dadurch gekennzeichnet**, das der Antrieb einen Kraftgang und einen Schnellgang aufweist, zwischen denen mechanisch oder elektrisch umgeschaltet werden kann.

11. Prothetische Greifeinheit nach einem der voranstehenden Ansprüche, **dadurch gekennzeichnet, dass** der Antrieb (4) von dem ersten Ausleger (10) entkoppelbar ist.

12. Prothetische Greifeinheit nach einem der voranstehenden Ansprüche, **dadurch gekennzeichnet, dass** an dem ersten Ausleger (10) und dem zweiten Ausleger (20) Halteeinrichtungen (11, 21) für ein Federelement (8) angeordnet sind.

13. Prothetische Greifeinheit nach einem der voranstehenden Ansprüche, **dadurch gekennzeichnet, dass** an einem dritten Ausleger (30) ein Betätigungshebel (9) gelagert ist, der über einen längsverschieblich gelagerten Bowdenzug (12) mit dem Träger der prothetischen Griffeinheit gekoppelt ist.

14. Prothetische Greifeinheit nach einem der voranstehenden Ansprüche, **dadurch gekennzeichnet, dass** eine Sensoreinheit (13) zur Erfassung des Betätigungsmomentes des Betätigungshebels (9) vorhanden ist.

15. Prothetische Greifeinheit nach einem der voranstehenden Ansprüche, **dadurch gekennzeichnet, dass** der erste Ausleger (10) über eine verlagerbare Verzahnung mit dem Antrieb (4) gekoppelt ist und die Verzahnung außer Eingriff mit dem Antrieb (4) bringbar ist.

## Claims

1. A prosthetic grip unit (1) with a base body (2) on which connection means (3) for attaching said grip unit to an upper extremity are arranged, with at least two members (10, 20) protruding from said base body, a first member (10) of the members being movably mounted such that relative to the second member (20) it can be displaced from a protruding into a flush position, and with the pivot axis (14) being aligned perpendicularly to the base body (2), wherein the first member (10) is driven by a motor, **characterized in that** the drive (4) is mounted in the base body (2) and that the first member (10) is mounted on the bearing (5) of the drive (4) and that an adjustment device (61, 62, 63, 65) for the closing force and/or speed of the pivot movement is arranged on the base body (2).

2. The prosthetic grip unit as claimed in one of the preceding claims, **characterized in that** a third member (30) is rigidly attached to the first member (10) or formed thereon.

3. The prosthetic grip unit as claimed in one of the preceding claims, **characterized in that** a receptacle (26) for a storage device (6) for electrical energy is arranged on the base body (2).

4. The prosthetic grip unit as claimed in one of the preceding claims, **characterized by** a myoelectric control of the drive (4).

5. The prosthetic grip unit as claimed in one of the preceding claims, **characterized in that** control electronics for the drive (4) are arranged in the base body (2).

6. The prosthetic grip unit as claimed in one of the preceding claims, **characterized in that** control electronics are arranged in the drive (4).

7. The prosthetic grip unit as claimed in one of the preceding claims, **characterized in that** the drive (4) is encapsulated in a waterproof fashion.

8. The prosthetic grip unit as claimed in one of the preceding claims, **characterized in that** the drive axle (14) of the drive (4) is oriented in parallel, in particular coaxially, to the pivot axis of the first member (10).

9. The prosthetic grip unit as claimed in one of the preceding claims, **characterized in that** an adjustment device (61, 62. 63, 65) for switching a mode is arranged on the base body (2).

10. The prosthetic grip unit as claimed in one of the preceding claims, **characterized in that** drive has a power gear and a speed gear, with it being possible to mechanically or electrically switch therebetween.

11. The prosthetic grip unit as claimed in one of the preceding claims, **characterized in that** the drive (4) can be decoupled from the first member (10).

12. The prosthetic grip unit as claimed in one of the preceding claims, **characterized in that** holding devices (11, 21) for a spring element (8) are arranged on the first member (10) and the second member (20).

13. The prosthetic grip unit as claimed in one of the preceding claims, **characterized in that** mounted on the third member (30) there is an actuation lever (9) which is coupled to the wearer of the prosthetic grip unit via a Bowden cable (12) mounted in a longitudinally displaceable fashion.

14. The prosthetic grip unit as claimed in one of the preceding claims, **characterized in that** there is a sensor unit (13) for detecting the actuation moment of the actuation lever (9).

15. The prosthetic grip0 unit as claimed in one of the preceding claims, **characterized in that** the first member (10) is coupled to the drive (4) via a displaceable toothing, and the toothing can be brought to disengage from the drive (4).

## Revendications

1. Unité de préhension prothétique (1) comprenant un corps de base (2), sur lequel sont agencés des moyens de raccordement (3) pour la fixation sur une extrémité supérieure, et duquel dépasse au moins deux bras (10, 20), parmi lesquels un premier bras (10) est monté de manière à pouvoir être déplacé par rapport au second bras (20) depuis une position en écartement jusque dans une position en contact, et l'axe de pivotement (14) est orienté perpendiculairement au corps de base (2), dans laquelle le premier bras (10) est entraîné de manière motorisée, **caractérisée en ce que** l'entraînement (4) est monté dans le corps de base (2), **en ce que** le premier bras (10) est monté sur le palier (5) de l'entraînement (4), et **en ce qu'**un moyen de réglage (61, 62, 63, 65) pour la force de fermeture et/ou pour la rapidité du mouvement de pivotement est agencé sur le corps de base (2).

2. Unité de préhension prothétique selon la revendication 1, **caractérisée en ce qu'**un troisième bras (30) est fixé de façon rigide sur le premier bras (10) ou conformé sur celui-ci.

3. Unité de préhension prothétique selon la revendication 1 ou 2, **caractérisée en ce qu'**un logement (26) pour un moyen d'accumulation (6) pour de l'énergie électrique est agencé sur le corps de base (2).

4. Unité de préhension prothétique selon l'une des revendications précédentes, **caractérisée par** un pilotage myoélectrique de l'entraînement (4).

5. Unité de préhension prothétique selon l'une des revendications précédentes, **caractérisée en ce qu'**un système électronique de commande pour l'entraînement (4) est agencé dans le corps de base (2).

6. Unité de préhension prothétique selon l'une des revendications précédentes, **caractérisée en ce qu'**un système électronique de commande est agencé dans l'entraînement (4).

7. Unité de préhension prothétique selon l'une des revendications précédentes, **caractérisée en ce que** l'entraînement (4) est encapsulé de manière étanche à l'eau.

8. Unité de préhension prothétique selon l'une des revendications précédentes, **caractérisée en ce que** l'axe d'entraînement (14) de l'entraînement (4) est orienté parallèlement et en particulier coaxialement à l'axe de pivotement du premier bras (10).

9. Unité de préhension prothétique selon l'une des revendications précédentes, **caractérisée en ce qu'**un moyen de réglage (61, 62, 63, 65) pour une commutation de mode est agencé sur le corps de base (2).

10. Unité de préhension prothétique selon l'une des revendications précédentes, **caractérisée en ce que** l'entraînement comprend une vitesse en force et une vitesse rapide, entre lesquelles il est possible de commuter par voie mécanique ou par voie électrique.

11. Unité de préhension prothétique selon l'une des revendications précédentes, **caractérisée en ce que** l'entraînement (4) est découplé vis-à-vis du premier bras (10).

12. Unité de préhension prothétique selon l'une des revendications précédentes, **caractérisée en ce que** des moyens de retenue (11, 21) pour un élément à ressort (8) sont agencés sur le premier bras (10) et sur le second bras (20).

13. Unité de préhension prothétique selon l'une des revendications précédentes, **caractérisée en ce qu'**un levier d'actionnement (9), qui est couplé via un câble Bowden (12) monté en translation longitudinale avec le support de l'unité de préhension prothétique, est monté sur un troisième bras (30).

14. Unité de préhension prothétique selon l'une des revendications précédentes, **caractérisée en ce qu'**il est prévu une unité sensorielle (13) pour détecter le couple d'actionnement du levier d'actionnement (9).

15. Unité de préhension prothétique selon l'une des revendications précédentes, **caractérisée en ce que** le premier bras (10) est couplé avec l'entraînement (4) via une denture déplaçable, et la denture est susceptible d'être amenée hors d'engagement avec l'entraînement (4).
